# EUROPEAN PATENT APPLICATION

(11) **EP 2 354 113 A1**
(43) Date of publication of application: **10.08.2011**
(21) Application number: 10001144.4
(22) Date of filing: 04.02.2010
(51) Int. Cl.: C07C 2/36, C08F 6/02, B01J 31/18, B01J 31/14

(54) **Method for deactivation of a catalyst**

(71) Applicant: Linde AG, 80331 München (DE)
(72) Inventor: Bölt, Heinz, 82515 Wolfratshausen (DE); Wellenhofer, Anton, 82069 Hohenschäftlarn (DE); Müller, Wolfgang, Dr., 81245 München (DE); Meiswinkel, Andreas, Dr., 81479 München (DE); Wöhl, Anina, 81379 München (DE); Harff, Marco, 81541 München (DE)
(74) Representative: Kasseckert, Rainer

(57) **Abstract**

The present invention relates to a method for deactivation of a catalyst which is suitable for catalyzing a chemical reaction, the catalyst being sensitive to oxygen and being present in a reaction medium comprising educt, product and optionally solvent, wherein the catalyst is treated with an oxidizing agent, introduced into the reaction medium for deactivation thereof.

## Description

The present invention relates to a method for deactivation of a catalyst.

Many chemical processes involving homogenous or heterogenous slurry-phase catalysts usually need some kind of quench of the reaction after a defined reaction time. This involves the controlled destruction or deactivation of the catalyst.

In particular, this step is necessary to prevent the reaction mass to undergo unwanted and uncontrolled continued reactions in the unit operations downstream of the reactor, giving rise to the formation of unwanted side products such as, e.g., heavies, waxes and polymers. Also, aggressive catalyst compounds may be incompatible with the materials in downstream equipment of a reactor, e.g., decanters, columns, vessels, reboilers, condensers, pumps, compressors, heat exchangers etc., and may therefore cause severe fouling- and/or corrosion problems.

For example, in typical olefin oligomerization reactions, the catalyst - separation and/or - deactivation is accomplished by distillation/evaporation of the products with subsequent spent catalyst incineration, by solvent washing procedures, by decomposition with aqueous acids or caustic solutions or via reaction with other compounds such as amines, alcohols, etc., see US 5,688,028 and US 7,476,775 B2.

In the prior art, the reaction solution (containing the product and the catalyst's constituents) is introduced into a water-, caustic- or acidic phase under inert atmosphere. Here, the underlying idea is to hydrolyze the catalyst components and to precipitate the metals of the catalytic complex and the ones of the co-catalyst. In the case of water or caustic, this leads to a precipitation of (often amphoteric) metal-oxides/-hydroxides, with the known disadvantages (solids handling, blockage/fouling of equipment, emulsion formation, handling-/separation of an additional aqueous phase, abrasion in pumps etc.). In principle, precipitation can largely be avoided by catalyst deactivation with aqueous acids but this causes material problems and possible corrosion.

A great disadvantage of homogeneous catalyst systems is that they are physically dissolved in the product stream, making it difficult to stop the reaction by separation of the catalyst from the reaction mass. However, in many cases it is necessary to terminate the catalytic reaction before the product is transferred to further process steps like separation section. Even if the target reaction is thermodynamically controlled, a well-defined kinetic control is necessary in most cases to minimize side reaction channels that are very often kinetically controlled. In a typical ethylene oligomerization reaction, for instance, a growing amount of branched products, double-bond isomerized products and side products, originating from reactions with the solvent, can lead to a severe deterioration of product quality if the reaction is not properly quenched after the required reaction time.

Furthermore, disparate process conditions may exist in the subsequent process steps, possibly causing unwanted and uncontrollable catalyzed reactions leading to fouling, runaway or otherwise uncontrollable conditions which have to be avoided, especially for safety reasons. Consequently, the homogenous catalyst has to be destroyed before the product can be treated further.

Very often this is accomplished by contacting the organic phase of the reaction mass with an aqueous phase containing either caustic or an acid. For example, an oligomerization process is known, wherein the catalyst is removed by extraction with caustic in a one step process, where the catalyst components are deactivated and transferred from the organic phase into the aqueous phase (see e.g. DE 198 07 226). This catalyst killing method may have under certain circumstances some disadvantages. For example, there is a possible chance of product degradation by unwanted side reactions during the catalyst - "killing" reaction. It is also possible that improper process conditions inadvertently lead to a backflow of the caustic into the reactor which has detrimental effects on the process. Also, the separation of the caustic from the product stream is costly and can be sensitive to variations of the process conditions e.g. in the mixers and decanters.

There is also a possibility to stop the reaction by adding an alcohol, e.g. ethanol or 2-ethyl-1-hexanol (US 5,689,028), to the product stream. However, a new chemical is introduced into the process and eventually the additive has to be separated by distillation. The additive has to be recycled or disposed which both requires additional investment efforts. Unfortunately, dissolved constituents of the destroyed catalyst system can contaminate the product leading to a significant loss of economic value. This is especially true in the case of light ethylene - oligomerization products which are used as co-monomers for, e.g., LLDPE-production (linear low density polyethylene). In these polymerization processes very high product purities are required to minimize detrimental effects on the polymerization reaction.

It is an object of the present invention to provide a method for deactivation of a catalyst which overcomes the drawbacks of the prior art. Especially, a method shall be provided wherein no additional aqueous phase has to be handled, like in quenching processes using aqueous acidic or caustic solutions. Further, the method shall provide a relatively simple control and easy separation of excess quenching medium and/or deactivated catalyst.

This object is achieved by a method for deactivation of a catalyst which is suitable for catalyzing a chemical reaction, the catalyst being sensitive to oxygen and being present in a reaction medium comprising educt, product and optionally solvent, wherein the catalyst is treated with an oxidizing agent, introduced into the reaction medium, for deactivation thereof.

It is preferred that the catalyst is an organic, inorganic or metalorganic catalyst, preferably a metalorganic catalyst.

More preferred, the catalyst is homogenous or heterogenous in a slurry phase.

In one embodiment of the invention, the oxidizing agent is selected from gaseous, liquid and/or solid oxidizing agents, preferably gaseous or dissolved air or oxygen.

Preferably, the oxidizing agent is used in a molar excess to the oxidizable catalyst, wherein after deactivation excess oxidizing agent is preferably removed.

In another embodiment of the invention, the residence time of catalyst containing reaction medium and oxidizing agent is from 1 to 180 minutes.

Most preferably, it is preferred that deactivated, preferably precipitated, catalytic components are removed from the reaction medium.

More preferred, the method is carried out in a deactivation vessel which is separated from a reactor for converting the chemical reaction.

Finally, it is preferred that the chemical reaction is oligomerization or polymerization of olefins, preferably of ethylene.

Surprisingly, it was found that the inventive method can be advantageously utilized in deactivating a catalyst which is suitable for catalyzing chemical reactions and which is sensitive to oxygen.

The method according to the invention is especially suitable for a broad range of homogeneously catalyzed reactions. The reason for this is that many homogeneous catalysts are, by their very nature, sensitive against oxidation, especially by dioxygen.

Depending on the nature of the particular process and its associated catalyst, the oxidation may occur either at the catalyst's active metal-center, the ligand or at the activator/co-catalyst. In most cases, all components of the catalytic system are simultaneously destroyed by oxidation.

For example, in polymerizations and oligomerizations, various types of aluminum-alkyls are being used as activators, e.g. methylaluminoxane (MAO), ethylaluminum sesquichloride, triethylaluminum, diethylaluminum chloride, etc.

It is known that sufficient, preferably excess amounts of molecular oxygen lead to total oxidation according to the following (exemplary, simplified) reactions equations:
Triethylaluminum (TEA):
   2 (CH₃CH₂)₃Al + 21 O₂→ 12 CO₂ + Al₂O₃ + 15 H₂O
Diethylaluminum chloride (DEAC):
   2 (CH₃CH₂)₂AlCl + 14 O₂ → CO₂ + Al₂O₃ + 2 HCl + 9 H₂O

The central idea of the invention is to take advantage of oxidative destruction in a very controlled way, namely by meticulously controlling the destruction-reaction kinetics in the liquid phase via suitable dosing of oxygen or other oxidants.

Furthermore, the reaction equations show that water is being formed during total oxidation of the aluminum-alkyls and, in the case of DEAC, even hydrochloric acid. These reaction products can cause the hydrolysis of typical ligands used in many homogeneously catalyzed reaction as a secondary "in-situ" deactivation process. The types of ligands typically used in such processes very often comprise, e.g., various alkylphosphines and/or phosphorous-nitrogen compounds such as, for example, bis-(diphenylphosphino)isopropylamine (or similarly substituted compounds).

The P-/N-based ligand "backbones" are known to be sensitive against oxygen (direct oxidation) and water/acid (secondary deactivation). In fact, many direct oxidation products of the phosphorous- and nitrogen have been analytically identified after exposure to oxygen.

Finally, the catalytic activitiy of many classes of catalyst systems relies on certain oxidation states (or the change of certain oxidation states during the catalytic reaction) of the catalyst's metal-center to which the ligand is coordinated.

In many cases, the catalytic activity of the metal center can simply be "switched off' by transferring the metal to a higher oxidation state.

In the inventive method no additional aqueous phase has to be handled, like in quenching processes using aqueous acidic or caustic solutions. Further, a relatively simple process control is possible through simple separation of the oxidizing agent, preferably air/oxygen. A simple separation of deactivated catalyst components, such as metal oxides in case of organometallic catalysts, or precipitated catalyst compounds due to their insolubility in organic solvents is possible. Further, the inventive method allows easy accumulation of expensive and polluting catalytic compounds for recycling, especially metal compounds; only small amounts have to be handled, as no additional solvent or water has to be utilized. This also improves the overall process sustainability by simple recycling of the catalytic compounds. Further, no unwanted side-reactions, catalyzed by catalyst components, are possible according to the inventive method. A further advantage relies in the fact that no contamination of the product stream by trace components from the catalyst deactivation reactions is provided. Moreover, no additional separation section recycle loop for additional catalyst-killing agents, like alcohols, amines, etc., is necessary. Finally, the product quality is not jeopardized by trace side reactions during catalytic deactivation, such as double-bond isomerisation, halogenation, hydration, etc.

In a preferred embodiment, if deactivation is carried out in a deactivation vessel, only a small deactivation vessel is necessary since no additional aqueous phase is used.

The present invention provides a method for deactivation of a catalyst by direct oxidation thereof.

The catalyst to be deactivated in the method of the present invention is to be understood as comprising an active catalytic species such as an organometallic catalytic complex, activators/co-catalysts and optional modifiers. In a most preferred embodiment, the catalyst is an organometallic catalyst.

Suitable oxidizing agents can be chosen from any chemical compounds, either liquid, gaseous or solid, which are able to release oxygen under in-situ conditions, thus oxidizing the catalyst. Suitable oxidizing agents may be therefore, for example, air, oxygen, peroxides or other solutions of organic/inorganic oxidizing salts.

Instead of gaseous air or oxygen, dissolved air or oxygen in a solvent can be applied as well. To accomplish this, the solvent can be pre-saturated with air/oxygen. This has the advantage that the kinetics of the gas-to-liquid phase transfer is decoupled from the kinetics of the oxidation. If the phase transfer is rate limiting, this may lead to a faster deactivation and consequently to a shorter residence time. Besides allowing for smaller deactivation vessels in a preferred embodiment, this can also contribute to the process control and improved inherent process safety, since the concentration of the oxidizing agent can be controlled and adjusted more easily.

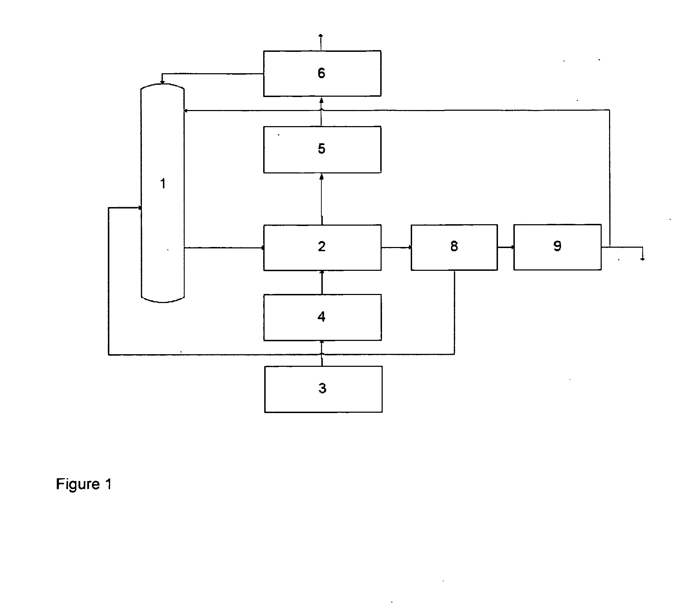

Additional features and advantages of the inventive method for deactivation of a catalyst can be taken from the following detailed description of a preferred embodiment with reference to the accompanying figure, which figure illustrates a flow sheet of a preferred embodiment of the inventive method.

The figure illustrates a reactor 1 in which educt is catalytically converted into product, optionally in the presence of an organic solvent. The reaction medium comprising educt, product, catalyst and optionally solvent is transferred from the reactor 1 to a deactivation vessel 2. Into that deactivation vessel 2, oxidizing agent, for example air or oxygen, is then introduced, preferably after having dried, filtrated and/or compressed a gaseous oxidizing agent in a drying and filtration step 3 and/or a compressing step 4.

The ratio of the oxidizing agent to the amount of catalyst is preferably adjusted so as to ensure that the entire catalyst, including all components, such as the active catalytic species, activators/co-catalysts and optional modifiers, are fully oxidized. Preferably, a small excess of oxidizing agent is used to ensure a complete catalyst deactivation.

The deactivation vessel 2 can, for example, be a stirred tank reactor, a bubble column or a ripple bed. To ensure a very good phase transfer, a sparger ring or a gas entrainment stirrer can be implemented. However, to ensure that the catalyst oxidation reaction is complete, a sufficient residence time is preferably to be assured. A preferred residence time is typically in the range of 1 minute to 180 minutes.

After oxidation of the catalyst in the deactivation vessel 2, the excess of (gaseous) oxidizing agent can be stripped off. Light products of the chemical reaction, for example 1-butene or 1-hexene in an oligomerization process of ethylene, as well as a light reactant (ethylene in the case of oligomerization) can be separated by condensation from that gaseous oxidizing agent stream, preferably in a separation step 5, whereafter educts can be preferably recirculated into the reactor 1 via a recirculation step 6. For safety reasons, the remaining gas stream leaving the recirculation step 6 which is not transferred to the reactor 1 is preferably piped to a flare.

The reaction medium comprising educt, product, deactivated catalyst and optionally solvent, can be transferred from the deactivation vessel 2 to a separation step 8. If the deactivated catalyst is present as precipitate, this precipitate can be easily separated from the reaction medium in step 8 by any means, such as filtration or the like. Depending on particle size distribution, viscosities and other physical properties of the deactivated catalyst products that are insoluble in the reaction medium, other separation techniques like cyclone, centrifuge or settling tanks can be used as well. Concentrated deactivated catalyst compounds, such as expensive metal compounds in the case of organometallic catalysts, can be recycled via further processing, leading to a good overall sustainability of the entire method. Furthermore the reaction medium (product stream) will not be contaminated by these compounds. If desired or suitable, the reaction medium which is freed of deactivated catalyst, can be recirculated to the reactor 1.

If residual dissolved oxidizing agent or oxygenates in the reaction medium interferes with downstream unit operations, for instance in the separation train, this should be also preferably removed, for example in a further separation step 9. This may be desirable in many cases, in particular for safety reasons. This removal of traces of oxidizing agent and oxygenates can be accomplished by a suitable adsorber. However, also other processes to eliminate oxidizing agent traces are conceivable, like, e.g. stripping with invert gases such as nitrogen.

The method for deactivation of a catalyst according to the present invention has been developed and can be preferably utilized in conjunction with a catalytic selective ethylene oligomerization technology (WO 2009/006979 A2 or WO 2009/068157 Al). However, it can be applied in any other processes using a homogenous or heterogenous (slurry phase) organic, inorganic or organometallic catalyst system which is sensitive to oxygen. Especially for organometallic catalysis, this is the case in practically all cases. A typical field of application of the oxidative catalyst -destruction/-deactivation is, e.g., the hydroformylation, especially of Cₙ long-chain -olefins to the corresponding Cₙ₊₁ - aldehydes and (after hydrogenation) Cₙ₊₁ -alcohols.

In these processes, the syngas (CO, H₂) is reacted with the olefins in liquid phase using, for instance, a "modified" cobalt - complex, where the ligand coordinated to the Co often comprises various trialkylphosphines. These catalytic complexes are susceptible to oxidative deactivation. Therefore, the invention can advantageously be applied for kinetic control, i.e. time-controlled quenching, of the hydroformylation reaction.

According to the invention is therefore also a process for catalytically converting educts to products, wherein that process comprises a method for deactivation of a catalyst as illustrated above.

The following detailed example is provided to illustrate the invention.

### Example

Oxidative deactivation of an ethylene trimerization catalyst system comprising chromium compound, an aminophosphine ligand with a PNPNH-backbone and triethylaluminum as cocatalyst. A catalyst system was formed in-situ, dissolved in toluene, as exemplified in WO 2009/006979 A2. In catalyst reference experiments in a stirred pressure reactor at 30 bar and 50-80°C, this ethylene trimerization system shows very high activities and selectivities towards 1-hexene formation.

After sparging the catalyst solution with air for 1 minute, small particles consisting of aluminum oxide and chromium oxide precipitated. After contacting the air-sparged catalyst solution with ethylene in a stirred pressure reactor at 30 bar and 50-80°C, absolutely no activity at all was observed. No ethylene consumption was observed and no 1-hexene product was formed under these conditions. Even unwanted side-reactions, like polymerization or wax formation, did not occur after this deactivation process.

The features disclosed in the foregoing description, in the claims and in the drawing may, both separately and in any combination thereof, be material for realizing the invention in diverse forms thereof.

## Claims

1. Method for deactivation of a catalyst which is suitable for catalyzing a chemical reaction, the catalyst being sensitive to oxygen and being present in a reaction medium comprising educt, product and optionally solvent, wherein the catalyst is treated with an oxidizing agent, introduced into the reaction medium, for deactivation thereof.

2. Method according to claim 1, wherein the catalyst is an organic, inorganic or metalorganic catalyst, preferably a metalorganic catalyst.

3. Method according to claim 1 or 2, wherein the catalyst is homogenous or heterogenous in a slurry phase.

4. Method according to any of the preceding claims, wherein the oxidizing agent is selected from gaseous, liquid and/or solid oxidizing agents, preferably gaseous or dissolved air or oxygen.

5. Method according to any of the preceding claims, wherein the oxidizing agent is used in a molar excess to the oxidizable catalyst, wherein after deactivation excess oxidizing agent is preferably removed.

6. Method according to any of the preceding claims, wherein residence time of catalyst containing reaction medium and oxidizing agent is from 1 to 180 minutes.

7. Method according to any of the preceding claims, wherein deactivated, preferably precipitated, catalytic components are removed from the reaction medium.

8. Method according to any of the preceding claims, wherein the method is carried out in a deactivation vessel which is separated from a reactor for converting the chemical reaction.

9. Method according to any of the preceding claims, wherein the chemical reaction is oligomerization or polymerization of olefins, preferably of ethylene.
